# EUROPEAN PATENT APPLICATION

(11) **EP 2 168 472 A2**
(43) Date of publication of application: **31.03.2010**
(21) Application number: 09011739.1
(22) Date of filing: 14.09.2009
(51) Int. Cl.: A61B 1/005, A61B 1/008, A61B 1/012

(54) **Endoscope having forceps channel**

(30) Priority: 25.09.2008 JP 2008245416; 25.09.2008 JP 2008245417; 25.09.2008 JP 2008245418
(71) Applicant: FUJIFILM Corporation, Minato-ku Tokyo (JP)
(72) Inventor: Sugisawa, Tatsuya, Saitama-shi Saitama (JP)
(74) Representative: Metzler, Volker

(57) **Abstract**

An endoscope of a transnasal type includes an elongated tube (12) for entry in a patient's body. A steering mechanism (20, 98, 105, 111, 114, 120, 125, 130, 135, 140, 145, 150) is provided in the elongated tube and on a proximal side from a distal end thereof, and bendable for changing a direction of the distal end. A forceps channel (16, 126) is formed through the elongated tube, for passing a treatment device to advance through the distal end. A constituent component (30, 62, 63, 85, 93, 94, 99-101, 112, 115a, 115b, 116, 146) is inserted in the elongated tube, disposed outside the forceps channel, and having an uneven thickness uneven within the steering mechanism in a direction transverse to an axial direction of the elongated tube, for extending in a tube lumen within the elongated tube. Specifically, the constituent component includes an air/water supply channel, or is an optical fiber bundle.

## Description

### BACKGROUND OF THE INVENTION

### 1. Field of the Invention

The present invention relates to an endoscope having a forceps channel. More particularly, the present invention relates to an endoscope which has a forceps channel, in which constituent components within an elongated tube can be free from buckling, and of which a steering mechanism can be operated easily.

### 2. Description Related to the Prior Art

An endoscope is used medically for diagnosis and treatment of a patient's body, and includes an elongated tube and a handle assembly. The elongated tube is entered in the body. The handle assembly is disposed at a proximal end of the elongated tube and manually operated by a doctor or operator. The elongated tube is cylindrical, has a length from several tens of centimeters to several meters, and has a diameter of several millimeters. In a proximal direction, the elongated tube includes a rigid assembly, a steering mechanism and a flexible portion.

The steering mechanism is constituted by plural steering ring-shaped link devices, which are coupled with one another in a rotatable manner for bending in one train. Four wire guide structures are disposed in each of the link devices and arranged in an arcuate direction. Each of the wire guide structures receives insertion of a pull strip of wire. Steering wheels are disposed on the handle assembly, and rotationally operated for bending the steering mechanism up or down, and to the right or left by moving the pull strip back and forth. Thus, the rigid assembly is directed as desired by the steering operation.

There are various constituent components inserted through the elongated tube, including a forceps channel, an air/water supply channel and light guides. The forceps channel is defined in a tube which extends from the handle assembly to the rigid assembly and has flexibility. A treatment device, such as forceps, is inserted in a first forceps opening of the forceps channel at the handle assembly, and comes to emerge through a second forceps opening of the forceps channel in a distal end face of the rigid assembly. The air/water supply channel is defined by its outer wall which extends inside a tube lumen from the handle assembly to the distal end face of the rigid assembly, has flexibility, and supplies air and water. The water is used for washing an imaging window for imaging of an object in the body. The air is used to blow away the water after use. Each of the light guides is a bundle of plural optical fibers, has a distal end positioned at a lighting window in the distal end face of the rigid assembly, and guides light from a light source for emission through the lighting window.

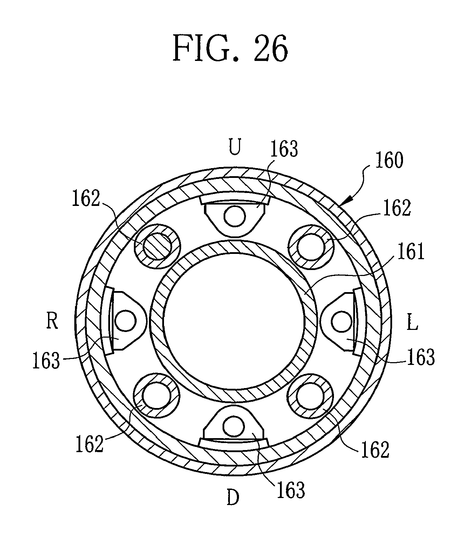

JP-A 10-033467 discloses an endoscope with a structure for preventing damage of a forceps channel due to load in bending a steering mechanism. In Fig. 26, the endoscope includes a steering mechanism 160 and a forceps channel 161 formed in a position at the center at least inside the steering mechanism 160. Four wire guide structures 163 are arranged in its tube lumen to regulate the forceps channel 161 at the center. Plural constituent components 162, such as an air/water supply channel, light guides and the like, are contained in regions split by the forceps channel 161 and the wire guide structures 163. Even upon bending the steering mechanism, the forceps channel 161 can remain at the center and the constituent components 162 can shift only in their regions. It is possible to prevent irregularity in the arrangement of the constituent components 162 with the forceps channel 161 and prevent damage due to interference between those elements.

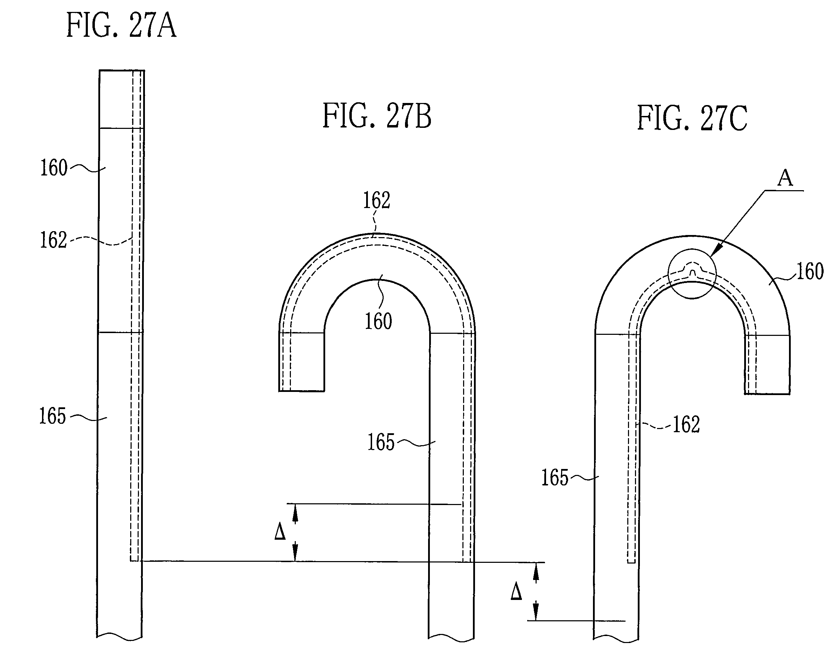

In Fig. 27A, an elongated tube 165 is illustrated with one of the constituent components 162 being conditioned. As the constituent components 162 are arranged in off-centered positions about the forceps channel 161, the constituent components 162 become positioned on one of external and internal sides with respect to the bent shape according to changes of directions of the steering mechanism 160 from the upside to the downside, or from the left to the right, or the like. In Fig. 27B, when the constituent component 162 is positioned on the external side with respect to the bent shape, a length of the constituent component 162 increases by a size Δ axially in the steering mechanism 160. In Fig. 27C, when the constituent component 162 is positioned on the internal side with respect to the bent shape, the length of the constituent component 162 decreases by the size Δ axially in the steering mechanism 160. Note that the sign Δ is not a specifically numerical expression, and but used for clarification with the drawings.

Considerable void spaces are present around each of the constituent components 162 as disclosed in the endoscope of JP-A 10-033467. See Fig. 26. One of the constituent components 162, when positioned on the internal side with respect to the bent shape, comes to flex within the steering mechanism 160 to absorb compression of the constituent components 162, as indicated by the arrow A in Fig. 27C. The constituent components 162 will be buckled upon flexing to the extent over the bending amount of the steering mechanism. If the light guide among the constituent components 162 is buckled, the optical fiber is folded. Thus, no light for illumination can be transmitted. If the air/water supply channel among the constituent components 162 is buckled, no air or water can be supplied.

An endoscope of transnasal type for an upper gastrointestinal tract has been developed recently, in which the elongated tube has a diameter smaller than that of an oral type of the endoscope, for example 6 mm or less, for the purpose of entry of the elongated tube through a patient's nostril. In a known model of the transnasal type of the endoscope, the forceps channel has a bore (for example, 2.0 mm) smaller than that of the forceps channel of the oral type of the endoscope (for example, 2.8 mm), for the purpose of reducing the diameter of the elongated tube. It is impossible in the transnasal type to utilize the treatment device designed for use in the oral type of the endoscope. Thus, a specialized type of the treatment device is constructed and used in combination with the transnasal type of the endoscope.

There is a new endoscope of the transnasal type in which a bore of the forceps channel is set as great as that of the endoscope of the oral type, for the purpose of utilizing even the treatment device originally produced for use with the endoscope of the oral type. In the endoscope, the diameter of the constituent components other than the forceps channel, for example the air/water supply channel and the light guides, is determined small, in order to keep a sufficient available region for containing the forceps channel. However, there arises a problem of buckling of the elongated tube due to drop of rigidity in reducing the diameter of the air/water supply channel, the light guides and the like.

In view of further reduction of the diameter of the elongated tube, it is important to dispose the constituent components at a very high density within the steering mechanism which has smaller available regions for enclosing the constituent components than other portions of the endoscope, because of disposition of the wire guide structures.

Also, the degree of freedom in changing the direction of the rigid assembly in the endoscope is high according to greatness of a maximum bending angle with which the steering mechanism can flex. A field of view of imaging can be enlarged. However, the greatness of the maximum bending angle of the steering mechanism creates a problem of quickening degradation of the constituent components because of their bending. In view of this, the endoscope of the known type is so constructed as to be bendable in one particular steering direction with the maximum bending angle of 180 degrees or more (for example, upward direction as viewed in a distal direction of imaging), and to be bendable in other steering directions with the maximum bending angle as small as 90 degrees.

According to greatness of the bending angle of the steering mechanism, rotational load to the link devices increases, and resistance to covering material around the link devices increases. Greater force is required for bending the steering mechanism. If the bending angle is at most 90 degrees, the load to operating the steering wheels at the handle assembly for bending the steering mechanism is equal between the four of the steering direction in a manner irrespective of disposition of the constituent components. Relatively small force is sufficient for bending. However, if the bending angle is more than 90 degrees, greater force will be required for bending due to higher load to operating the steering wheels. There arises a problem of drop in the operability in steering.

### SUMMARY OF THE INVENTION

In view of the foregoing problems, an object of the present invention is to provide an endoscope which has a forceps channel, in which constituent components within an elongated tube can be free from buckling, and of which a steering mechanism can be operated easily.

In order to achieve the above and other objects and advantages of this invention, an endoscope includes an elongated tube for entry in a body. A steering mechanism is provided in the elongated tube and on a proximal side from a distal end thereof, and bendable for changing a direction of the distal end. A forceps channel is formed through the elongated tube, for passing a treatment device to advance through the distal end. A constituent component is inserted in the elongated tube, disposed outside the forceps channel, and has an uneven thickness uneven within the steering mechanism in a direction transverse to an axial direction of the elongated tube, for extending in a tube lumen within the elongated tube.

A thickness of the constituent component in an arcuate direction of a sectional shape of the elongated tube is greater than a thickness of the constituent component in a radial direction of the elongated tube.

The constituent component has an elliptic sectional shape, which has a long axis positioned to extend in the arcuate direction of the sectional shape of the elongated tube, and a short axis positioned to extend in the radial direction of the elongated tube.

A thickness of the constituent component is uneven within the steering mechanism in a direction transverse to the axial direction of the elongated tube, and a sectional shape of the constituent component gradually changes from a portion on a proximal side from the steering mechanism to the steering mechanism.

The forceps channel is disposed to extend on an axis of the elongated tube at least within the steering mechanism. The constituent component is contained in a region defined by an inner wall of the steering mechanism and an outer wall of the forceps channel.

Furthermore, plural wire guide structures are disposed along the inner wall of the steering mechanism in an axial direction of the elongated tube, for receiving insertion of plural pull strips for bending the steering mechanism upon pull on a proximal side of the elongated tube. The constituent component is contained in one of plural regions defined by splitting with the inner wall of the steering mechanism, the outer wall of the forceps channel, and the plural wire guide structures.

At least one of the regions is a void region without containing the constituent component.

The steering mechanism is bendable in plural steering directions with different bending angles. The void region is positioned in one of the steering directions where the bending angle is greatest.

The steering mechanism is bendable in plural steering directions with different bending angles. One of the regions is positioned in one of the steering directions where the bending angle is greatest, and contains a constituent component having a smaller flexural rigidity than flexural rigidity of the constituent component contained in a remaining one of the regions.

The wire guide structures are four wire guide structures arranged equidistantly in the arcuate direction of the elongated tube, and the regions are four regions. The steering mechanism is bendable in plural steering directions being upward, downward, rightward and leftward, and a bending angle in the upward steering direction is greatest among bending angles in the steering directions.

The constituent component includes a channel lumen formed through.

The constituent component includes an air/water supply channel for supply air or water through the channel lumen. A cross-sectional area of a portion of the channel lumen other than the steering mechanism is 1.1-3 times as large as a cross-sectional area of the channel lumen within the steering mechanism.

Furthermore, plural branch conduits are defined by splitting the channel lumen in a position short of the steering mechanism, for extending through the steering mechanism.

The constituent component includes covering material disposed on a surface thereof for wrapping.

The constituent component has an approximately circular sectional shape as viewed in a cross section relative to the axial direction of the elongated tube, and the covering material has an uneven thickness uneven in the direction transverse to the axial direction.

Furthermore, plural branch portions are defined by splitting the constituent component in a position short of the steering mechanism, for extending through the steering mechanism.

The branch portions of the constituent component are wrapped by the covering material commonly together or discretely from one another.

The constituent component is an optical fiber bundle, supplied with laser light of semiconductor laser from a light source, for guiding the laser light to phosphor for emitting white light upon excitation with the laser light.

The optical fiber bundle is wrapped in the covering material being single together with another constituent component.

The constituent component is one of a water jet tube adapted to water jet treatment, and a zooming pull strip for zooming of an imaging optical system incorporated at the distal end.

Furthermore, a tensioning mechanism pulls the constituent component toward a proximal end of the elongated tube.

Also, an endoscope is provided, and includes an elongated tube for entry in a body. A steering mechanism is provided in the elongated tube and on a proximal side from a distal end thereof, and bendable for changing a direction of the distal end. Plural wire guide structures are disposed along an inner wall of the steering mechanism in an axial direction of the elongated tube, for receiving insertion of plural pull strips for bending the steering mechanism upon pull on a proximal side of the elongated tube. A forceps channel is formed through the elongated tube, disposed to extend on an axis thereof at least within the steering mechanism, for passing a treatment device to advance through the distal end. A constituent component is inserted in the elongated tube, and contained in one of plural regions defined by splitting with the inner wall of the steering mechanism, an outer wall of the forceps channel, and the plural wire guide structures, wherein at least a portion of the constituent component is contained in the steering mechanism and has an elliptic sectional shape, which has a long axis positioned to extend in an arcuate direction of a sectional shape of the elongated tube, and a short axis positioned to extend in a radial direction of the elongated tube.

Also, an endoscope includes an elongated tube for entry in a body. A steering mechanism is provided in the elongated tube and on a proximal side from a distal end thereof, and bendable for changing a direction of the distal end. Plural wire guide structures are disposed along an inner wall of the steering mechanism in an axial direction of the elongated tube, for receiving insertion of plural pull strips for bending the steering mechanism upon pull on a proximal side of the elongated tube. A forceps channel is formed through the elongated tube, disposed to extend on an axis thereof at least within the steering mechanism, for passing a treatment device to advance through the distal end. A constituent component is inserted in the elongated tube, and contained in one of plural regions defined by splitting with the inner wall of the steering mechanism, an outer wall of the forceps channel, and the plural wire guide structures. At least one of the regions is a void region without containing the constituent component, or contains a constituent component having a smaller flexural rigidity than flexural rigidity of the constituent component contained in a remaining one of the regions.

The endoscope is a transnasal type for entry of the elongated tube through a nostril. The forceps channel is a type adapted to a transoral endoscope.

Specifically, the steering mechanism includes first to Nth link devices arranged in a train in an axial direction toward a proximal side. A dual axis joint device is provided intermediately within the train of the first to Nth link devices, for supporting the first link device in a manner rotatable about a first axis transverse to the axial direction, and rotatable about a second axis transverse to the axial direction and to the first axis.

The dual axis joint device includes a first pivot structure for supporting a (2K) th one of the link devices on a (2K-1) th one thereof in a rotatable manner about the first axis, where K is an integer in a range from 1 to N/2. A second pivot structure supports a (2K+1)th one of the link devices on the (2K)th link device in a rotatable manner about the second axis.

Accordingly, constituent components within an elongated tube can be free from buckling, and of which a steering mechanism can be operated easily, because the constituent component has the uneven thickness within the steering mechanism transversely to the axial direction of the elongated tube.

### BRIEF DESCRIPTION OF THE DRAWINGS

The above objects and advantages of the present invention will become more apparent from the following detailed description when read in connection with the accompanying drawings, in which:
Fig. 1 is a front elevation illustrating appearance of an endoscope;
Fig. 2 is a plan illustrating a distal end face of a rigid assembly;
Fig. 3 is a horizontal section illustrating a steering mechanism;
Fig. 4 is an exploded perspective view illustrating link devices of one train in the steering mechanism;
Fig. 5 is a cross section illustrating a flexible portion;
Fig. 6 is a cross section illustrating the steering mechanism;
Fig. 7 is a horizontal section illustrating a shape of a constituent component within the steering mechanism;
Fig. 8 is an explanatory view illustrating a tensioning mechanism for light guides;
Fig. 9 is a cross section illustrating a shape of the constituent component for use in CAE analysis;
Fig. 10 is a perspective view illustrating appearance of the constituent component for use in the CAE analysis;
Fig. 11 is a graph illustrating a relationship between a long axis length of the constituent component and a buckling radius;
Fig. 12 is a horizontal section illustrating a model for use in the CAE analysis of compressive buckling;
Fig. 13 is a table illustrating results of analyzing the compressive buckling;
Fig. 14A is a cross section illustrating another preferred constituent component with a sectional shape of an arcuate form;
Figs. 14B and 14C are cross sections illustrating still other preferred constituent components;
Fig. 15 is a cross section illustrating still another preferred steering mechanism;
Fig. 16 is a cross section illustrating the steering mechanism including single covering material for wrapping signal lines and optical fiber bundles;
Fig. 17 is a cross section illustrating the steering mechanism in which two branch lumens extends from an air/water supply channel;
Fig. 18 is a cross section illustrating the steering mechanism including single covering material for wrapping two optical fiber bundles of light guides;
Fig. 19 is a cross section illustrating the steering mechanism of which a third region is void;
Fig. 20 is a cross section illustrating the steering mechanism in which an optical fiber bundle is contained in a forceps channel;
Fig. 21 is a cross section illustrating the steering mechanism of which a first region is void;
Fig. 22 is a cross section illustrating another preferred steering mechanism of which a first region is void;
Fig. 23 is a cross section illustrating the steering mechanism of which a light guide is positioned on an upper side;
Fig. 24 is a cross section illustrating the steering mechanism including a water jet tube;
Fig. 25 is a cross section illustrating the steering mechanism including a zooming pull strip;
Fig. 26 is a cross section illustrating a structure of the steering mechanism according to prior art;
Figs. 27A, 27B and 27C are front elevations illustrating shifts and shifting directions of the constituent component upon bending the steering mechanism.

### DETAILED DESCRIPTION OF THE PREFERRED EMBODIMENT(S) OF THE PRESENT INVENTION

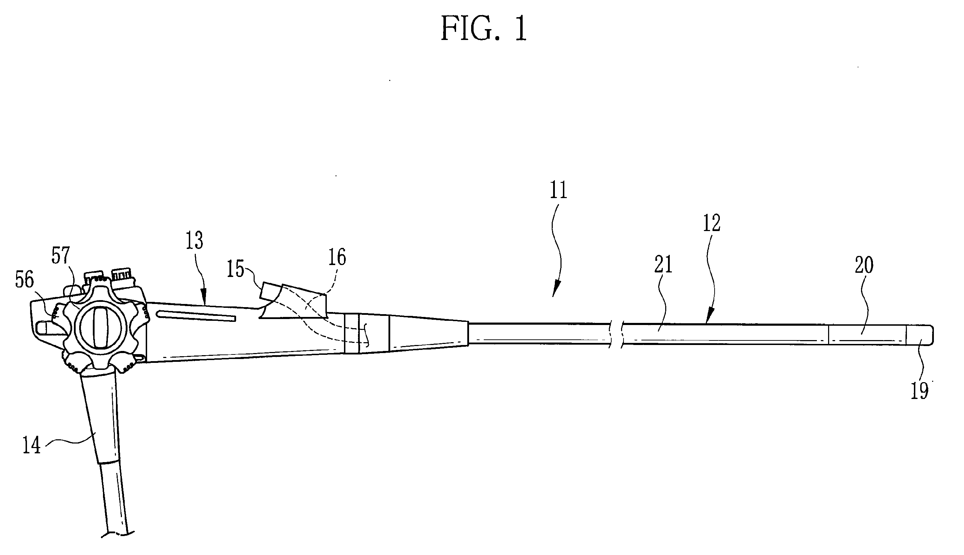

In Fig. 1, an endoscope 11 is a transnasal type for a gastrointestinal tract. The endoscope 11 includes an elongated tube 12 or insertion tube, a handle assembly 13 and a universal cable 14. The elongated tube 12 is entered in a patient's body through his or her nose. The handle assembly 13 is positioned at a proximal end of the elongated tube 12. The universal cable 14 is connected with the handle assembly 13. A first forceps opening 15 is formed in the handle assembly 13 for insertion of a treatment device for endoscopic access to a body part. A forceps channel 16 is formed in the elongated tube 12, is defined in a tubular outer wall, and extends from the first forceps opening 15 as indicated by the broken line.

The elongated tube 12 is cylindrical, and has a diameter of approximately 6 mm and a length of 1-2 meters. The elongated tube 12 includes a rigid assembly 19 or head assembly, a steering mechanism 20 and a flexible portion 21 arranged in a proximal direction.

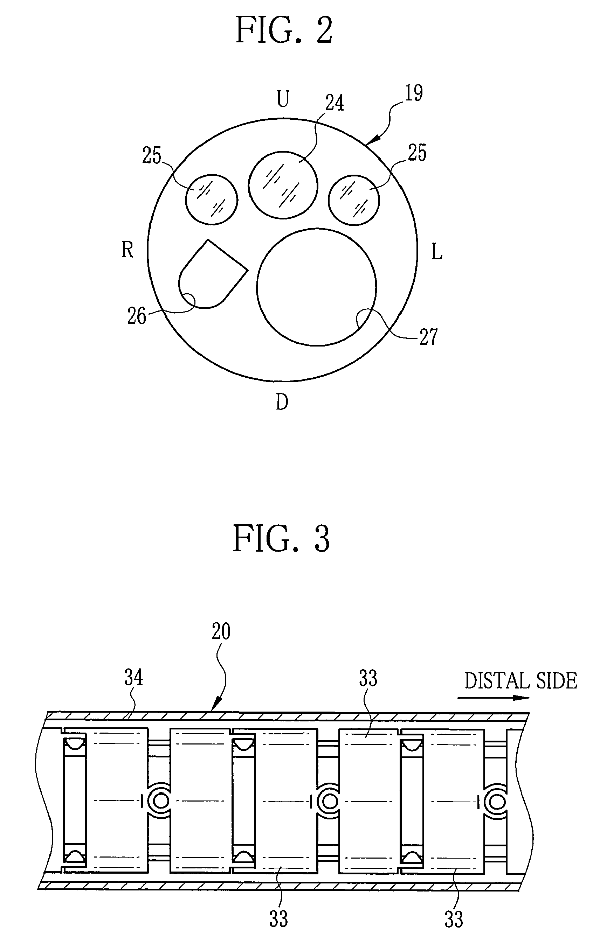

In Fig. 2, a distal end face of the rigid assembly 19 is circular. An imaging window 24 is formed in the center of the distal end face on the upper side, and receives image light of an image from an object. Two lighting windows 25 are disposed beside the imaging window 24, and apply light of illumination to the object. A transparent panel is fixedly mounted in each of the imaging window 24 and the lighting windows 25. An air/water supply nozzle 26 is positioned on the left side and lower than the imaging window 24, and ejects air or water to the imaging window 24 for washing. A second forceps opening 27 is formed in the distal end face, is positioned on the right lower side, is connected with the forceps channel 16, and causes a distal end of a treatment device to emerge forwards. In the drawing, reference signs U, D, L and R arranged about the rigid assembly 19 designate directions of bending the steering mechanism 20, namely the upward, downward, left and right sides.

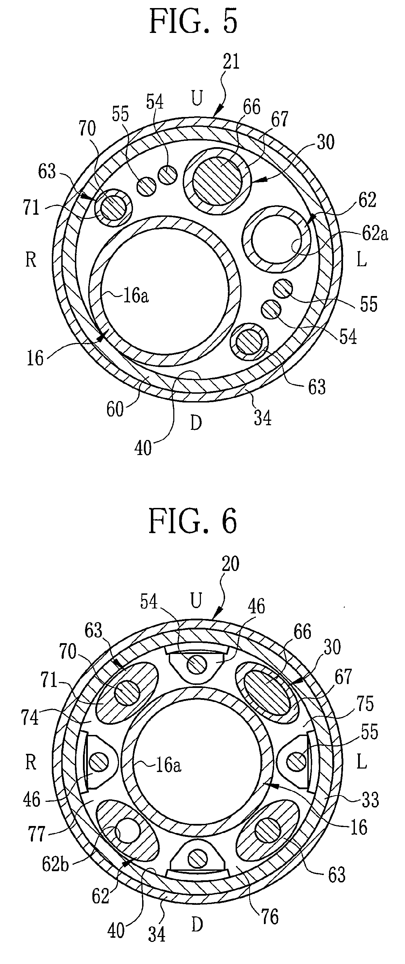

A lens system and an image pickup device (not shown), included in the head assembly, are positioned inside the imaging window 24. The image pickup device outputs an image signal by picking up an image of an object focused by the lens system through the imaging window 24. A signal cable 30 extends as illustrated in Fig. 5 and is inserted through the elongated tube 12, the handle assembly 13 and the universal cable 14. The image signal is sent by the signal cable 30 to a processor (not shown). An image of the image signal is processed by the processor in image processing of various functions, and is displayed on a monitor display panel (not shown).

In Fig. 3, plural steering link devices 33 of one train for a form of a link chain are included in the steering mechanism 20, are associated with a dual axis joint device, and operate for steering by bending. Covering material 34 or sleeve material of a flexible property is applied to wrap the outside of the link devices 33. A first one (not shown) of the link devices 33 is fixedly secured to the rigid assembly 19. A final one (not shown) of the link devices 33 is fixedly secured to the flexible portion 21.

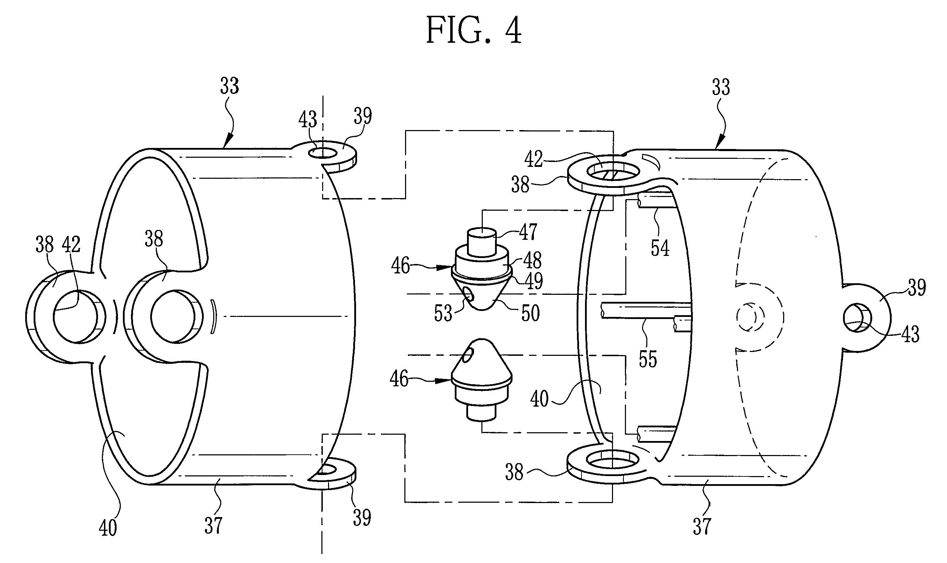

In Fig. 4, each one of the link devices 33 includes an articulating ring 37 for the form of the link chain, and an inner pivot wheel 38 and outer pivot wheel 39 for the link connection. The inner pivot wheel 38 projects from a distal end of the ring 37. The outer pivot wheel 39 projects from a proximal end of the ring 37, and is positioned to receive the inner pivot wheel 38.

A pivot hole 42 for the link connection is formed at the center of the inner pivot wheel 38. The outer pivot wheel 39 has a smaller size than the inner pivot wheel 38. A small pivot hole 43 for the link connection is formed in the outer pivot wheel 39, and has a smaller size than the pivot hole 42. Groups of the inner pivot wheel 38 and the outer pivot wheel 39 are arranged alternately with a difference of 90 degrees in the arcuate direction of the ring 37, in a form constituting a dual axis joint device, the groups being arranged in the axial direction. The inner pivot wheel 38 is offset internally from the outer pivot wheel 39 in the radial direction of the ring 37. An amount of the offsetting is as much as the wall thickness of the ring 37.

A pivot pin 46 for the link connection couples two of the link devices 33 with one another. The pivot pin 46 includes a pin rod 47 with a small diameter, an intermediate portion 48 with a large diameter, a pin head 49, and a tapered wire guide structure 50. The tapered wire guide structure 50 has a rounded end, and has a conical form which is distinct from cylindrical forms of the pin rod 47, the intermediate portion 48 and the pin head 49.

In the pivot pin 46, the pin rod 47 is inserted in the small pivot hole 43 and the intermediate portion 48 is inserted in the pivot hole 42 after overlapping the outer pivot wheel 39 of one of the link devices 33 on the inner pivot wheel 38 of a second one of the link devices 33 on the proximal side. An end face of the intermediate portion 48 is set on an inner face of the outer pivot wheel 39, to couple the link devices 33 with one another in a rotatable manner. After the coupling between the link devices 33, the rear end of the pin rod 47 is caulked to keep the pivot pin 46 on the link devices 33 without drop. A height of the intermediate portion 48 is greater than a height of the inner pivot wheel 38. A gap is formed between the inner and outer pivot wheels 38 and 39 and between the inner pivot wheel 38 and the pin head 49 to enable the link devices 33 smoothly to rotate on the proximal side.

A guide hole 53 is formed in the tapered wire guide structure 50 to extend in its radial direction. There are pull strips 54 of wire for vertical steering, and pull strips 55 of wire for horizontal steering. Each of the pull strips 54 and 55 is inserted through the guide hole 53. In Fig. 1, the handle assembly 13 has a vertical steering wheel 56 and a horizontal steering wheel 57. Pulleys (not shown) are disposed in the handle assembly 13. The vertical steering wheel 56 is rotatable together with a first one of the pulleys. The horizontal steering wheel 57 is rotated together with a second one of the pulleys. Each of the pull strips 54 and 55 has first and second ends. The first end is fixedly secured to the rigid assembly 19. The pull strip 54 or 55 extends through a tube lumen 40 in the steering mechanism 20 and the flexible portion 21, and is in contact with one of the pulleys to extend in reverse by use of the periphery of the pulleys. The second end is also fixedly secured to the rigid assembly 19. When the vertical steering wheel 56 is rotated, the pull strips 54 are moved back or forth. When the horizontal steering wheel 57 is rotated, the pull strips 55 are moved back or forth. The link devices 33 of the steering mechanism 20 rotate about their coupled pivot portions in response to shift of the pull strips 54 or 55. Thus, the direction of the rigid assembly 19 is changed.

In Fig. 5, the flexible portion 21 includes a tubular coil 60 of an elongate strip of metal, and the covering material 34 of resin disposed about the tubular coil 60. An air/water supply channel 62 is formed through the elongated tube 12 having the flexible portion 21. Two light guides 63 are inserted through the flexible portion 21 as well as the forceps channel 16, the signal cable 30 and the pull strips 54 and 55.

The forceps channel 16 is disposed on the right side in the flexible portion 21 in a lower position as viewed in the distal direction related to the steering. An example of the outer wall of the forceps channel 16 is a tube molded from plastic material, silicone resin or the like having a watertight and flexible property. A channel lumen 16a of the forceps channel 16 has a bore of 2.8 mm. This size is greater than a bore 2.0 mm of a forceps channel of a known endoscope of a transnasal type, and approximately equal to a diameter of forceps channel of a transoral type of endoscope. Thus, the structure of the forceps channel 16 is effective in a combined use of a treatment device of a transoral endoscope by safe insertion.

The signal cable 30 is disposed in the flexible portion 21 and higher than the forceps channel 16. The signal cable 30 includes plural signal lines 66 and covering material 67 or sleeve material disposed about the signal lines 66 by resin forming. An example of the covering material 67 is plastic material, silicone resin or the like having a watertight and flexible property.

The air/water supply channel 62 is disposed on the left side from the forceps channel 16 in the flexible portion 21 and higher than the forceps channel 16, and disposed on the left side from the signal cable 30 and lower than the signal cable 30, as viewed in the distal direction related to the steering. An example of the outer wall of the air/water supply channel 62 is molded from plastic material, silicone resin or the like having a watertight and flexible property. A channel lumen 62a is defined in the air/water supply channel 62, and has a circular shape as viewed in cross section with a smallest loss in consideration of reduced resistance to flow of air and water. The air/water supply channel 62 extends from the air/water supply nozzle 26 through the handle assembly 13 to the universal cable 14, and is connected with a pump (not shown) for supply air and water. The air/water supply channel 62 supplies the air/water supply nozzle 26 with air and water from the pump.

The light guides 63 cause light to pass for illuminating the object. The light guides 63 are disposed in the tube lumen 40 and between an inner wall of the flexible portion 21 and an outer wall of the forceps channel 16 so as to locate the forceps channel 16 between those. Each of the light guides 63 includes material. The optical fiber bundle 70 has a circular sectional shape of which an outer diameter is approximately 0.1-0.2 mm. The covering material 71 is applied to wrap the outside of the optical fiber bundle 70 by resin forming. An example of the covering material 71 is plastic material, silicone resin or the like having a watertight and flexible property.

A proximal end of the optical fiber bundle 70 is inserted through the handle assembly 13 into the universal cable 14, and is connected with a light source device (not shown). A light source is incorporated in the light source device for emitting blue laser light of semiconductor laser through the proximal end of the optical fiber bundle 70. A distal end of the optical fiber bundle 70 is coated with phosphor (not shown) which is excited by the blue laser light and emits white light. The phosphor is positioned inside the lighting windows 25 in the rigid assembly 19. The white light travels through the lighting windows 25 toward an object of interest.

An example of light source for illumination in the present embodiment is a laser white light source including a laser device of a semiconductor laser for emitting blue light, and phosphor, such as Micro White (trade name) manufactured by Nichia Chemical Industries, Ltd. The Micro White can be combined with the optical fiber bundle with the diameter of 0.16 mm to produce light at a light amount substantially equal to that available from a known light source of a xenon lamp with the optical fiber bundle of a diameter of approximately 1.2 mm. The use of the Micro White is effective in reducing the diameter of the endoscope 11 and also maintaining light of a sufficient light amount.

The pull strips 54 and 55 extend within the flexible portion 21. One of the pull strips 54 and one of the pull strips 55 are positioned between the signal cable 30 and the light guide 63. A remaining one of the pull strips 54 and a remaining one of the pull strips 55 are positioned between the air/water supply channel 62 and the light guide 63.

In Fig. 6, the forceps channel 16 is formed at the center of the steering mechanism 20, and regulated by four pivot pins 46 arranged at a rotational angle of 90 degrees. There are a first region 74, a second region 75, a third region 76 and a fourth region 77 arranged about the forceps channel 16 and separated by the pivot pins 46 in the tube lumen 40. Constituent components other than the forceps channel 16 are contained in the first to fourth regions 74-77. The light guides 63 are contained in respectively the first region 74 and the third region 76 positioned opposite to the first region 74. The signal cable 30 is contained in the second region 75. The air/water supply channel 62 is contained in the fourth region 77.

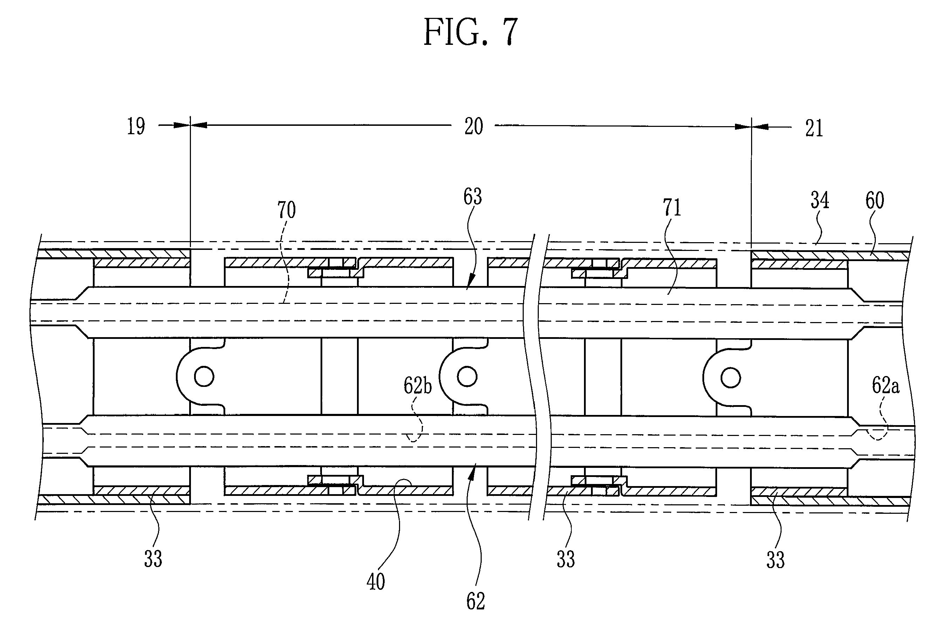

Shapes of the covering material 67 of the signal cable 30, the covering material 71 of the light guides 63 and the air/water supply channel 62 as viewed in a cross section are elliptic in such a manner that its long axis extends in the arcuate direction of the circumference of the steering mechanism 20 and its short axis extends in the radial direction of the steering mechanism 20. Those shapes are in compliance with the arcuate forms of the first to fourth regions 74-77. In Fig. 7, portions of the covering material 71, the air/water supply channel 62 and the covering material 67 (not shown) inside the rigid assembly 19 and the flexible portion 21 have a circular shape as viewed in a cross section. In contrast, portions of the covering materials 67 and 71 and the air/water supply channel 62 inside the steering mechanism 20 have an elliptic shape as viewed in the cross section. In Fig. 7, the forceps channel 16, the signal cable 30, the pivot pin 4 6 and the pull strips 54 and 55 are not depicted for the purpose of clarifying the air/water supply channel 62 and the light guides 63 in the elongated tube 12.

When the steering mechanism 20 is bent, the constituent component in the elongated tube 12 shifts axially. In view of this, sectional shapes of the covering materials 67 and 71 and the air/water supply channel 62 gradually change from the circle to the ellipse without an abrupt change at the boundary of the steering mechanism 20. This is for the purpose of preventing interference of the covering materials 67 and 71 and the air/water supply channel 62 during their axial shift within the elongated tube 12. In Fig. 27B, one of the constituent components on an outer side in bending of the steering mechanism 20 is introduced therein. Even portions of the covering materials 67 and 71 and the air/water supply channel 62, which may be contained in the flexible portion 21 with the straight shape of the steering mechanism 20, have elliptic shapes as viewed in a cross section, inclusive of parts to be introduced in the steering mechanism 20 during its bending. In relation to the boundary between the rigid assembly 19 and the steering mechanism 20, details are the same as those related to the boundary between the flexible portion 21 and the steering mechanism 20.

The signal lines 66 are positioned within the covering material 67 in an elliptic shape in the steering mechanism 20 with a difference from the inside of the flexible portion 21. A channel lumen 62b and the optical fiber bundle 70 have a circular shape as viewed in a cross section. The channel lumen 62b has a smaller width than the channel lumen 62a in the flexible portion 21. In short, the covering materials 67 and 71 and the air/water supply channel 62 have an elliptic shape as viewed in a cross section, and have an uneven thickness in a direction perpendicular to the axial direction of the elongated tube 12. A thickness of the covering materials 67 and 71 and the air/water supply channel 62 have a greater first thickness in the arcuate direction of the steering mechanism 20 than a second thickness in the radial direction. In the embodiment, a ratio of the first thickness of those in the arcuate direction to the second thickness is approximately three (3). Note that the thickness ratio can be changed suitably according to the substance of the constituent components, diameter and other property.

In the air/water supply channel 62, the channel lumen 62b of the steering mechanism 20 has a smaller diameter than the channel lumen 62a extending in the rigid assembly 19 and the flexible portion 21 as described above. A ratio of a cross-sectional area of the channel lumen 62a to that of the channel lumen 62b is equal to or greater than 1.1 and equal to or smaller than 3. This can minimize the pressure drop while a flow path of air or water is maintained sufficiently for requirement even when the diameter of the channel lumen 62b is determined according to the space of the steering mechanism 20.

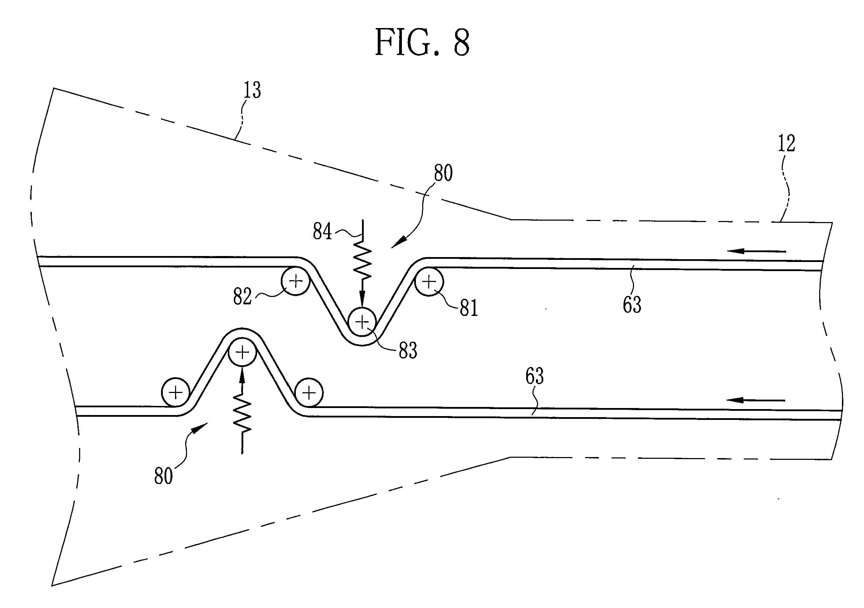

In Fig. 8, a tensioning device 80 is contained in the handle assembly 13 and pulls the light guides 63 toward the handle assembly 13. The tensioning device 80 includes a pair of guide rollers 81 and 82, a tension roller 83 and a spring 84. The light guides 63 extend in contact with the guide rollers 81 and 82. The tension roller 83 is movable in a direction perpendicular to the axial direction of the light guides 63 between the guide rollers 81 and 82. The spring 84 biases the tension roller 83 in a direction to apply tension to the light guides 63. When any of the light guides 63 is positioned on the inner side according to the curved shape and is ready to flex with compression in the axial direction (See Fig. 27C), the tension roller 83 is shifted in a direction to absorb the flexing by the bias of the spring 84.

The operation of the above embodiment is described now. At first, the elongated tube 12 of the endoscope 11 is entered through a nostril of a patient into a gastrointestinal tract for endoscopic examination. The vertical and horizontal steering wheels 56 and 57 are operated as required for steering, to move the pull strips 54 and 55 back or forth in the elongated tube 12. The link devices 33 in the steering mechanism 20 rotate pivotally according to shift of the pull strips 54 and 55 to change the direction of the rigid assembly 19 or head assembly by bending.

The forceps channel 16 is disposed at the center of the steering mechanism 20. As the tube lumen 40 contains constituent components in the first to fourth regions 74-77 around the forceps channel 16, bending of the steering mechanism 20 can be smooth. There is no excessive load to the forceps channel 16 when the steering mechanism 20 is bent. Also, the forceps channel 16 and other constituent components are mounted at a high density to reduce gaps within the steering mechanism 20, constituent components in the steering mechanism 20 can be kept tightly and regularly. It is possible to prevent the constituent components from breakage even upon a random shift of those because the constituent components are kept from moving radially. The constituent components can be prevented from buckling even upon bending.

The constituent components including the air/water supply channel 62 and the light guides 63 within the steering mechanism 20 are arranged about the forceps channel 16 or the center. In Fig. 27C, any of the constituent components disposed on an inner side of the bend compresses in the axial direction when the steering mechanism 20 is bent.

The constituent components such as the signal cable 30, the air/water supply channel 62 and the light guides 63 in the steering mechanism 20 have the elliptic forms in compliance with the first to fourth regions 74-77 of the forceps channel 16. Shiftable ranges of the constituent components are kept small in the arcuate directions in the steering mechanism 20. Thus, the constituent components can be free from buckling, because there is no space of allowing flexing the constituent components in the steering mechanism 20 even upon its compression in the axial direction.

The thickness of the covering materials 67 and 71 and the air/water supply channel 62 is so uneven as to be greater in an arcuate direction of the steering mechanism 20 than in its radial direction. Thus, the constituent components can have high rigidity and can be free from buckling upon bending the steering mechanism 20.

Also, the tensioning device 80 applies tension to the light guides 63 in the proximal direction. The light guides 63 can be prevented from buckling within the steering mechanism 20, so as to transmit light reliably by avoiding folding of the optical fiber.

### EXAMPLES

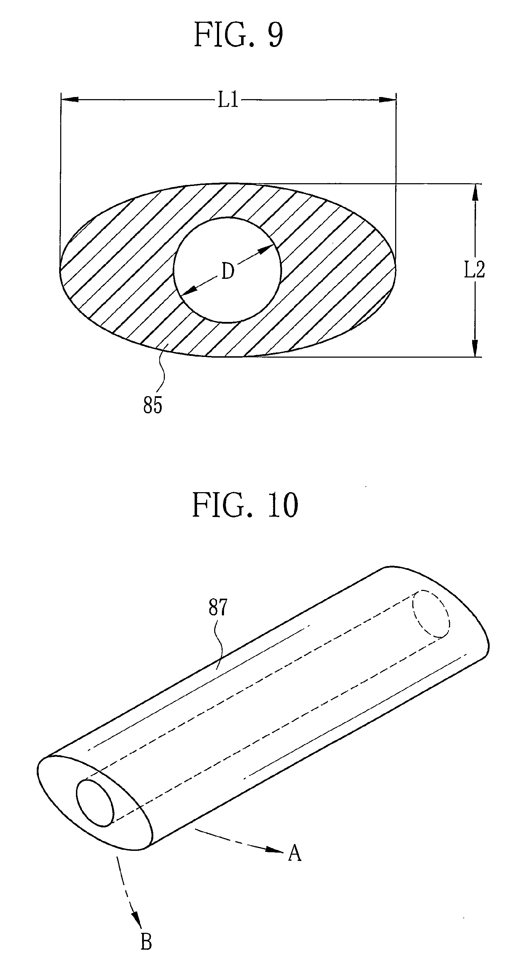

A CAE (Computer Aided Engineering) analysis was conducted to check a relationship between buckling and a sectional shape of a constituent component. In Fig. 9, a constituent component 85 had an elliptical sectional shape in a similar manner to the above embodiment in consideration of a void region in the steering mechanism 20. The following were the sizes and material of the constituent component 85, and a minimum bend radius of the steering mechanism. Note that the minimum bend radius of the steering mechanism was a radius of curvature of a curved line defined by the steering mechanism upon its maximum bending.

Bore D = 0.6 mm
Length of a long axis L1 = 1.7 mm or less
Length of a short axis L2 = 0.9 mm
Material: polytetrafluoroethylene (PTFE)

Minimum bend radius of the steering mechanism: approximately 6 mm.

A relationship between the bend radius of the constituent component 85 and occurrence of buckling was observed according to assumption that buckling of the constituent components was local buckling caused by their flexing together with the steering mechanism. Specifically, a CAE model 87 (Computer Aided Engineering model) of Fig. 10 was produced by running a computer according to the sectional shape of the constituent component 85. A bend radius (buckling radius) of occurrence of buckling was obtained by bending in the CAE model 87 in the steering direction A along the long axis and in the steering direction B along the short axis. The buckling radius was a bend radius at the time that a torque as reaction to flexing of the CAE model 87 reached its peak.

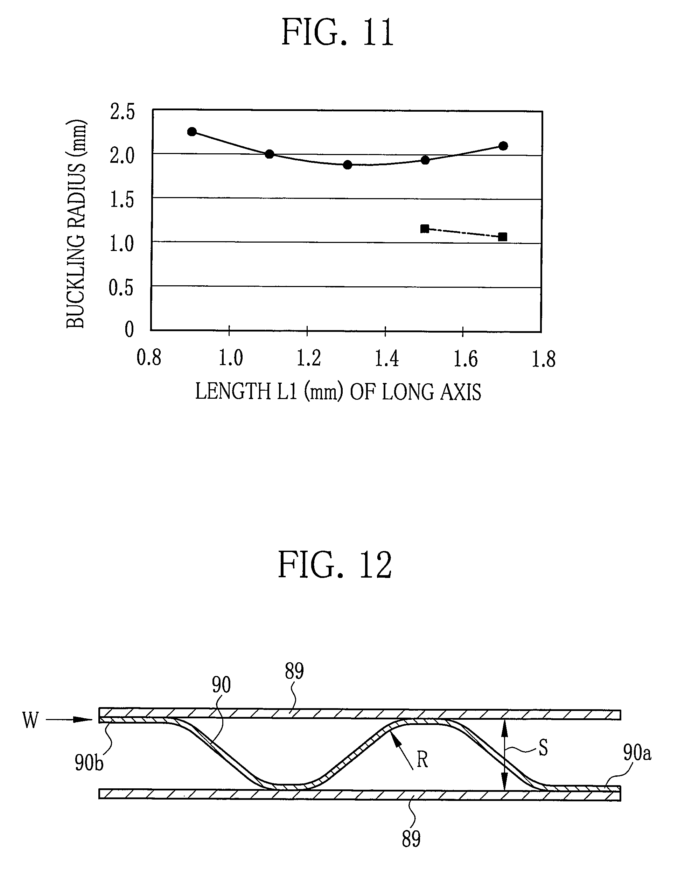

In Fig. 11, the buckling radii in the directions A and B were illustrated with changes in the long axis of the CAE model 87 in the range of 0.9-1.7 mm. A curve indicated by the solid line was obtained according to the direction A. A curve indicated by the broken line was obtained according to the direction B. Smallness in the buckling radius is an expression of small possibility of the buckling even upon flexing at a small angle. In short, the buckling radius of the constituent component 85 was greater in the direction A than in the direction B. The constituent component 85 was less resistant to sharp bending in the direction A than in the direction B. However, the buckling radii in both of the directions A and B were considerably smaller than 6 mm or the minimum bend radius of the steering mechanism. No buckling occurred even when the steering mechanism was bent with the minimum bend radius. As a result, the buckling created in the constituent component 85 was supposed not to be local buckling due to bending of the steering mechanism according to the initial assumption, but to be compressive buckling in which the constituent component 85 compressed in the axial direction within the elongated tube 12 upon bending the steering mechanism and buckled by local absorption of the compression in a manner of bending.

In the constituent component 85, resistance to the buckling was found the highest when the length L1 of the long axis was 1.3-1.4 mm in the graph for the steering direction A and when the length L1 of the long axis was 1. 7 mm in the graph for the steering direction B. It is thus concluded that a preferable value of the length L1 of the long axis is 1.7 mm. This is because a void or space for the constituent component 85 in the steering mechanism should be reduced to avoid flexing of the constituent component 85 to prevent buckling, and because a change in the radius of buckling is as small as 0.1-0.9 mm and considerably smaller than a bend radius of the steering mechanism.

Then the CAE analysis was conducted according to the compressive buckling of the constituent component 85. A size of a gap about the constituent component 85 was changed. The force of pressing sufficient for reaching the above-described buckling radius, at the time that the constituent component 85 was axially pressed in, was obtained by the analysis. Specifically, a pair of rigid bodies 89 and a cantilever beam 90 of Fig. 12 were utilized. The rigid bodies 89 were disposed at a space S. The cantilever beam 90 was inserted between the rigid bodies 89 in a curved manner for contact with those. The use of the cantilever beam 90 was based on regarding the constituent component 85 as a beam. A first end 90a of the cantilever beam 90 was fixedly secured. A second end 90b of the cantilever beam 90 was pressed into a position between the rigid bodies 89 with force W. The force W at the time of reach of the bend radius R of the cantilever beam 90 to the buckling radius was obtained. Examples of the cantilever beam 90 for use were Samples A and B. Sample A was the constituent component 85 with a sectional shape of Fig. 9. Sample B was a component in a known endoscope with occurrence of buckling, and had a circular sectional shape having an outer diameter of 0.9 mm and bore of 0.6 mm.

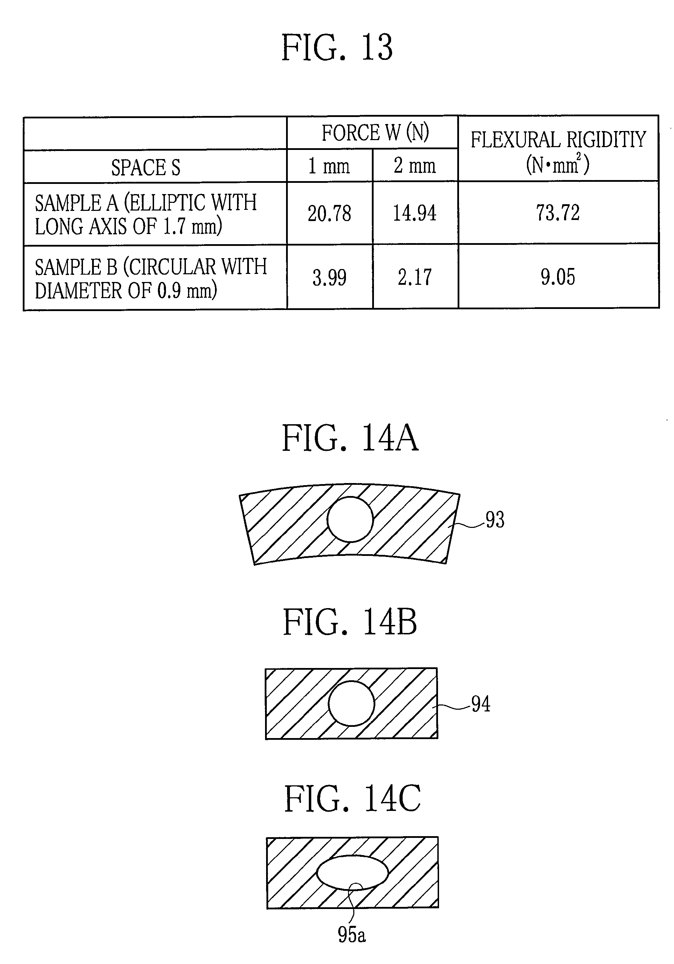

The table of Fig. 13 indicates the flexural rigidity and the force W of pressing for bending the cantilever beam 90 to the buckling radius with the space S of either 1 or 2 mm. When the space S was 2 mm in contrast with 1 mm, buckling occurred with lower force W of pressing. Thus, reduction of looseness in the tube lumen 40 containing the constituent component 85 was found effective in preventing buckling. As the sectional shape of the constituent component was elliptic with a long axis as long as 1.7 mm in contrast with a circular shape with a diameter of 0.9 mm, the thickness was uneven to raise flexural rigidity. Resistance to buckling, or force W of pressing required for buckling the constituent component, was 5-7 times as high for the sample A as for the sample B. It is concluded that occurrence of buckling can be suppressed by use of the constituent component with unevenness of the thickness in the sectional shape according to the observation related to Fig. 11 specifically for compressive buckling as one main cause of buckling of the constituent component.

The flexural rigidity of the constituent component is sufficiently smaller than that of the entirety of the steering mechanism 20. Load to operation of the steering mechanism for bending is mainly derived from factors such as force for bending the covering material 34, or the like other than the constituent component itself. There is no drop of the operability of the endoscope 11 even with higher flexural rigidity of the constituent component.

In the above embodiments, the constituent component has an elliptic shape as viewed in a cross section. However, a constituent component may have any suitable shape of an uneven thickness as viewed in a cross section so as to obtain higher rigidity. In Fig. 14A, a preferred constituent component 93 is illustrated, for example, with an air/water supply channel, and has a sectional shape of an arcuate form of the first to fourth regions 74-77. In Fig. 14B, a constituent component 94 is illustrated, and has a quadrilateral form. The channel lumen of the air/water supply channel is preferably circular when viewed in a cross section in consideration of resistance to flow of air and water. However, a channel lumen 95a may be formed in a manner of Fig. 14C, for example, an elliptic shape different from a circular shape for the purpose of enlarging a cross-sectional area in consideration of pressure drop. Note that a constituent component may be different from the examples in Figs. 14A-14C. For example, a constituent component may be a signal cable, light guide and the like, and can be a combination including an inner structure and a covering material formed about the inner structure.

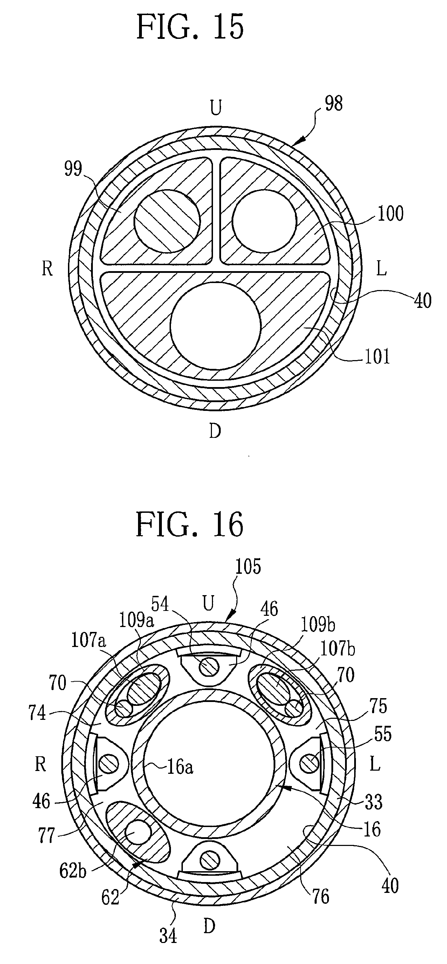

An optical fiber bundle for a white light laser light source has as small a diameter as 0.1 mm. It is conceivable to cover the optical fiber bundle together with a signal cable by use of a common covering material. In such a structure, main constituent components in the elongated tube are only the forceps channel, the signal cable and the air/water supply channel. In Fig. 15, one preferred steering mechanism 98 is illustrated, in which constituent components 99, 100 and 101 or fillers have sectional shapes for filling an entire gap within the steering mechanism 98. For example, the constituent component 99 corresponds to a signal cable or light guide. The constituent components 100 and 101 correspond respectively to an air/water supply channel and forceps channel. It is possible to prevent buckling because higher rigidity can be obtained by uneven thickness with constituent components of such sectional shapes. Note that the pivot pin for the link connection is not depicted in Fig. 15.

In Fig. 16, a steering mechanism 105 is illustrated. Signal lines 107a and 107b of two channels are inserted in the steering mechanism 105 as branch portions from the flexible portion to the boundary between the steering mechanism and the rigid assembly. Covering material 109a or sleeve material is applied to wrap the signal line 107a and the optical fiber bundle 70 of the light guide as a common covering. Also, covering material 109b or sleeve material is applied to wrap the signal line 107b and the optical fiber bundle 70 of the light guide as a common covering. The optical fiber bundle 70 may be structured in various forms, for example, may be constituted by helically winding the signal lines 107a and 107b, or simply by arranging the signal lines 107a and 107b side by side in the drawing. Handling of the optical fiber bundle 70 without other support would be extremely difficult due to its small thickness. However, the optical fiber bundle 70 can be handled with considerable ease in the embodiment because wrapped together with the signal lines 107a and 107b. Wrapping together of the signal lines 107a and 107b and the optical fiber bundle 70 makes it possible to set the third region 76 as a void region unlike the above embodiment in which the light guides 63 are contained in the third region 76.

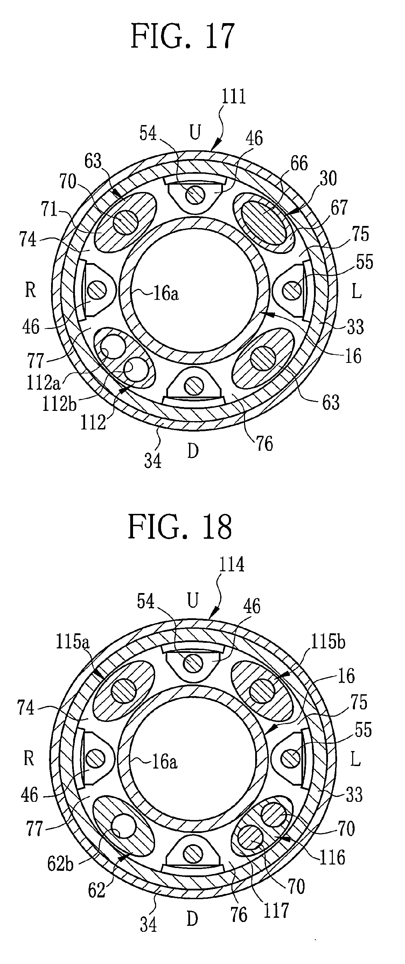

In Fig. 17, a steering mechanism 111 is illustrated. There is an air/water supply channel 112 in the steering mechanism 111, in which two channel lumens 112a and 112b are formed as branch lumens split from the flexible portion to a boundary between the steering mechanism and the rigid assembly. Note that it is preferable to set the cross-sectional area of the air/water supply channel in portions other than the steering mechanism 1. 1-3 times as large as the cross-sectional area of the air/water supply channel in the steering mechanism.

In Fig. 18, a steering mechanism 114 is illustrated, and includes a covering material 117 or sleeve material, a light guide 116 and signal cables 115a and 115b. The covering material 117 is single and wraps the periphery of the light guide 116. An original signal line is split to extend the signal cables 115a and 115b as branch portions. This is effective in enlarging a thickness of a covering material for the signal cables 115a and 115b in comparison with the above embodiment. Durability of the signal cable can be high.

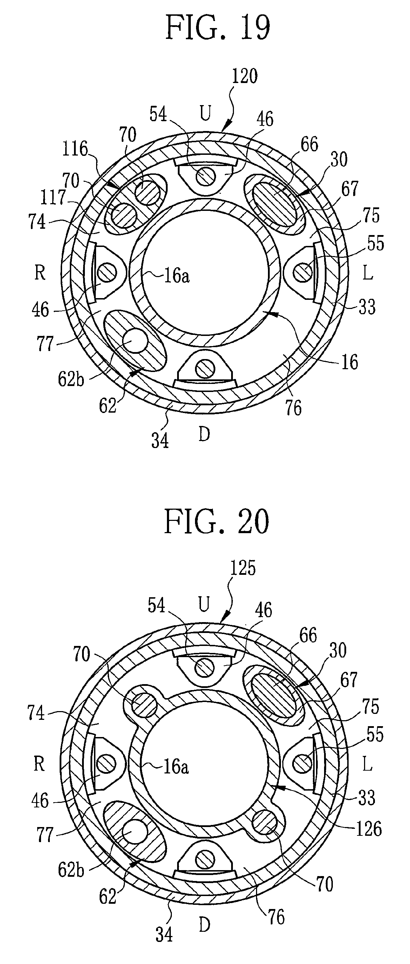

In Fig. 19, a steering mechanism 120 includes the signal cable 30 of Fig. 6 in the manner of the steering mechanism 20 and also the light guide 116 of Fig. 18. The third region 76 can remain as a void region. In Fig. 20, a steering mechanism 125 has a forceps channel 126 in which the optical fiber bundles 70 are enclosed.

The maximum bending angle of the steering mechanism 20 may be uneven between directions. For example, the maximum bending angle may be 210 degrees in the upward direction, 100 degrees in each of the lateral directions, and 90 degrees in the downward direction. Load to the vertical steering wheel 56 in the operation may be specifically high in the steering in the upward direction with a bending angle over 90 degrees. Thus, a void region is kept without containing a constituent component among the first to fourth regions 74-77 in the examples of Figs. 16, 19 and 20. The void region is positioned on the side of the upward direction where the load to the vertical steering wheel 56 may be high.

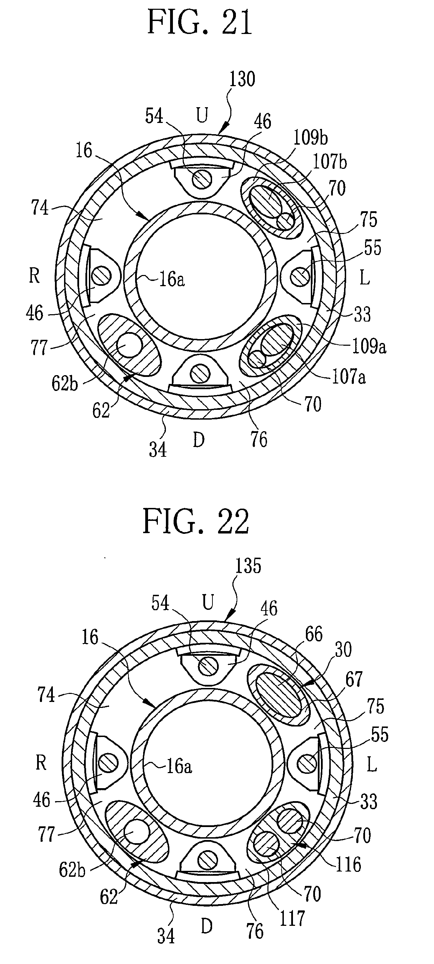

In Fig. 21, a steering mechanism 130 has an internal structure equal to that of the steering mechanism 105 of Fig. 16, but has a difference in that the first region 74 is void on the upper side and that the third region 76 is used for containment. In Fig. 22, a steering mechanism 135 has an internal structure equal to that of the steering mechanism 120 of Fig. 19, but has a difference in that the first region 74 is void on the upper side.

Load to the steering wheels in the course of bending the steering mechanism is equal between the steering directions if the bending angle is 90 degrees or less. If the steering mechanism is bent at more than 90 degrees and also in an upward direction for the maximum bending angle, then there occur increases in resistance to rotation of the link devices and resistance to bending of the covering material. In view of this, the steering mechanisms 130 and 135 of Figs. 21 and 22 have the first region 74 of a maintained void for facilitating bending by reducing upward resistance to the bending. The steering wheels can be free from an excessive increase in the load to rotation. Thus, the operability can be high as load in the manual operation for bending the steering mechanism can be regulated at a constant level.

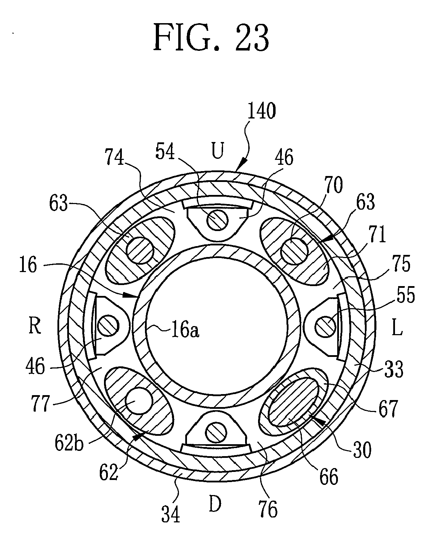

In Fig. 23, a steering mechanism 140 is illustrated as a variant of the steering mechanism 20 of Fig. 6. The signal cable 30 and the air/water supply channel 62 are contained in respectively the third and fourth regions 76 and 77. The light guides 63 are contained in respectively the first and second regions 74 and 75. The optical fiber bundle 70 for a laser white light source has a very small width and has small flexural rigidity. Unlike the structures of Figs. 21 and 22 with a void region in the first region 74, the first and second regions 74 and 75 are loaded with a constituent component having a smaller flexural rigidity such as the light guides 63 than other constituent components. This is also effective in reducing load to manual operation for the purpose of bending the steering mechanism in the upward direction.

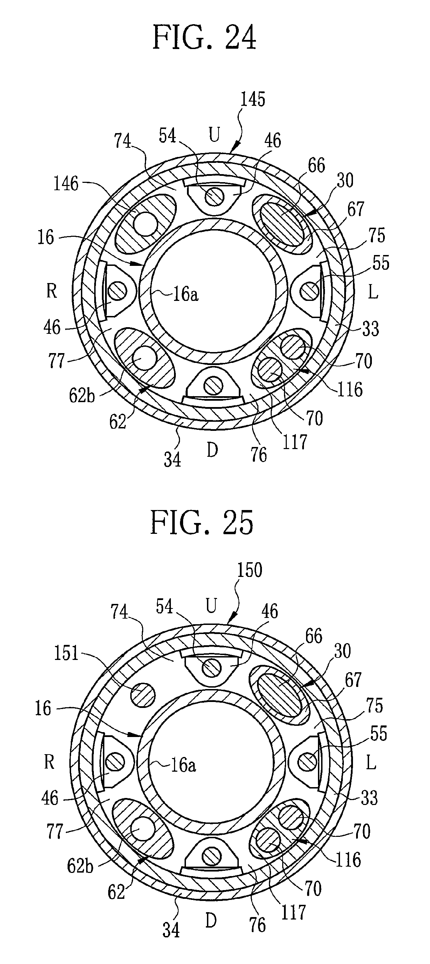

Furthermore, additional constituent components can be enclosed in a steering mechanism by locally tightening regions of the above-described constituent components. In Fig. 24, a steering mechanism 145 is illustrated, in which a water jet tube 146 is contained and the first region 74 is set as a void region. The water jet tube 146 is for use with a water jet scaplel device. In Fig. 25, another preferred steering mechanism 150 is illustrated. A zooming pull strip 151 of wire is contained in the first region 74 for operating a zoom lens which constitutes the lens system for imaging in the rigid assembly.

In each of the embodiments, only one of the split regions is void. However, two or more void regions may be formed. A position of a void region may be determined in any one direction different from the direction of the maximum bending angle.

In the embodiments, the tensioning device is associated only with the light guide. However, a tensioning device can be additionally associated with an outer wall of the air/water supply channel to apply tension. In the embodiments, the laser light source of white light is used. However, an endoscope of the invention may be used with a halogen lamp as light source well-known in the art.

In the embodiments, the sectional shape of the constituent components is elliptic only in the steering mechanism. However, parts of the constituent components in the flexible portion or rigid assembly may have an elliptic shape as viewed in a cross section. A sectional shape of the constituent components may be circular specifically when those with a small void region or small flexural rigidity are disposed in a steering direction of the maximum bending angle for the purpose of reducing load to manual operation. Furthermore, a position of the forceps channel may be offset from the axis at the center.

Furthermore, an endoscope of the invention may be a type other than the above transnasal type, for example, a transoral endoscope for use by swallowing, a colonoscope for a lower gastrointestinal tract, and the like.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope comprising:
an elongated tube (12) for entry in a body;
a steering mechanism (20, 98, 105, 111, 114, 120, 125, 130, 135, 140, 145, 150), provided in said elongated tube and on a proximal side from a distal end thereof, and bendable for changing a direction of said distal end;
a forceps channel (16, 126), formed through said elongated tube, for passing a treatment device to advance through said distal end;
a constituent component (30, 62, 63, 85, 93, 94, 99-101, 112, 115a, 115b, 116, 146), inserted in said elongated tube, disposed outside said forceps channel, and having an uneven thickness uneven within said steering mechanism in a direction transverse to an axial direction of said elongated tube, for extending in a tube lumen within said elongated tube.

2. An endoscope as defined in claim 1, wherein a thickness of said constituent component in an arcuate direction of a sectional shape of said elongated tube is greater than a thickness of said constituent component in a radial direction of said elongated tube.

3. An endoscope as defined in claim 2, wherein said constituent component has an elliptic sectional shape, which has a long axis positioned to extend in said arcuate direction of said sectional shape of said elongated tube, and a short axis positioned to extend in said radial direction of said elongated tube.

4. An endoscope as defined in claim 2, wherein a thickness of said constituent component is uneven within said steering mechanism in a direction transverse to said axial direction of said elongated tube, and a sectional shape of said constituent component gradually changes from a portion on a proximal side from said steering mechanism to said steering mechanism.

5. An endoscope as defined in claim 2, wherein said forceps channel is disposed to extend on an axis of said elongated tube at least within said steering mechanism;
said constituent component is contained in a region defined by an inner wall of said steering mechanism and an outer wall of said forceps channel.

6. An endoscope as defined in claim 5, further comprising plural wire guide structures, disposed along said inner wall of said steering mechanism in an axial direction of said elongated tube, for receiving insertion of plural pull strips for bending said steering mechanism upon pull on a proximal side of said elongated tube;
wherein said constituent component is contained in one of plural regions defined by splitting with said inner wall of said steering mechanism, said outer wall of said forceps channel, and said plural wire guide structures.

7. An endoscope as defined in claim 6, wherein at least one of said regions is a void region without containing said constituent component.

8. An endoscope as defined in claim 7, wherein said steering mechanism is bendable in plural steering directions with different bending angles;
said void region is positioned in one of said steering directions where said bending angle is greatest.

9. An endoscope as defined in claim 6, wherein said steering mechanism is bendable in plural steering directions with different bending angles;
one of said regions is positioned in one of said steering directions where said bending angle is greatest, and contains a constituent component having a smaller flexural rigidity than flexural rigidity of said constituent component contained in a remaining one of said regions.

10. An endoscope as defined in claim 6, wherein said wire guide structures are four wire guide structures arranged equidistantly in said arcuate direction of said elongated tube, and said regions are four regions;
said steering mechanism is bendable in plural steering directions being upward, downward, rightward and leftward, and a bending angle in said upward steering direction is greatest among bending angles in said steering directions.

11. An endoscope as defined in claim 1, wherein said constituent component includes a channel lumen formed through.

12. An endoscope as defined in claim 11, wherein said constituent component includes an air/water supply channel for supply air or water through said channel lumen;
a cross-sectional area of a portion of said channel lumen other than said steering mechanism is 1.1-3 times as large as a cross-sectional area of said channel lumen within said steering mechanism.

13. An endoscope as defined in claim 11, further comprising plural branch conduits, defined by splitting said channel lumen in a position short of said steering mechanism, for extending through said steering mechanism.

14. An endoscope as defined in claim 1, wherein said constituent component includes covering material disposed on a surface thereof for wrapping.

15. An endoscope as defined in claim 14, wherein said constituent component has an approximately circular sectional shape as viewed in a cross section relative to said axial direction of said elongated tube, and said covering material has an uneven thickness uneven in said direction transverse to said axial direction.

16. An endoscope as defined in claim 14, further comprising plural branch portions, defined by splitting said constituent component in a position short of said steering mechanism, for extending through said steering mechanism.

17. An endoscope as defined in claim 16, wherein said branch portions of said constituent component are wrapped by said covering material commonly together or discretely from one another.

18. An endoscope as defined in claim 14, wherein said constituent component is an optical fiber bundle, supplied with laser light of semiconductor laser from a light source, for guiding said laser light to phosphor for emitting white light upon excitation with said laser light.

19. An endoscope as defined in claim 18, wherein said optical fiber bundle is wrapped in said covering material being single together with another constituent component.

20. An endoscope as defined in claim 1, wherein said constituent component is one of a water jet tube adapted to water jet treatment, and a zooming pull strip for zooming of an imaging optical system incorporated at said distal end.

21. An endoscope as defined in claim 1, further comprising a tensioning mechanism for pulling said constituent component toward a proximal end of said elongated tube.

22. An endoscope comprising:
an elongated tube (12) for entry in a body;
a steering mechanism (20, 105, 111, 114, 120, 125, 130, 135, 140, 145, 150), provided in said elongated tube and on a proximal side from a distal end thereof, and bendable for changing a direction of said distal end;
plural wire guide structures (50), disposed along an inner wall of said steering mechanism in an axial direction of said elongated tube, for receiving insertion of plural pull strips (54, 55) for bending said steering mechanism upon pull on a proximal side of said elongated tube;
a forceps channel (16, 126), formed through said elongated tube, disposed to extend on an axis thereof at least within said steering mechanism, for passing a treatment device to advance through said distal end;
a constituent component (30, 62, 63, 85, 112, 115a, 115b, 116, 146), inserted in said elongated tube, and contained in one of plural regions (74-77) defined by splitting with said inner wall of said steering mechanism, an outer wall of said forceps channel, and said plural wire guide structures, wherein at least a portion of said constituent component is contained in said steering mechanism and has an elliptic sectional shape, which has a long axis positioned to extend in an arcuate direction of a sectional shape of said elongated tube, and a short axis positioned to extend in a radial direction of said elongated tube.

23. An endoscope comprising:
an elongated tube (12) for entry in a body;
a steering mechanism (20, 105, 111, 114, 120, 125, 130, 135, 140), provided in said elongated tube and on a proximal side from a distal end thereof, and bendable for changing a direction of said distal end;
plural wire guide structures (50), disposed along an inner wall of said steering mechanism in an axial direction of said elongated tube, for receiving insertion of plural pull strips (54, 55) for bending said steering mechanism upon pull on a proximal side of said elongated tube;
a forceps channel (16, 126), formed through said elongated tube, disposed to extend on an axis thereof at least within said steering mechanism, for passing a treatment device to advance through said distal end;
a constituent component (30, 62, 63, 112, 116), inserted in said elongated tube, and contained in one of plural regions (74-77) defined by splitting with said inner wall of said steering mechanism, an outer wall of said forceps channel, and said plural wire guide structures;
wherein at least one of said regions is a void region without containing said constituent component, or contains a constituent component having a smaller flexural rigidity than flexural rigidity of said constituent component contained in a remaining one of said regions.

24. An endoscope as defined in claim 23, wherein said endoscope is a transnasal type for entry of said elongated tube through a nostril;
said forceps channel is a type adapted to a transoral endoscope.
